# EUROPÄISCHE PATENTSCHRIFT

(11) **EP 3 392 342 B1**
(45) Veröffentlichungstag und Bekanntmachung des Hinweises auf die Patenterteilung: **12.06.2019**
(21) Anmeldenummer: 18161905.7
(22) Anmeldetag: 15.03.2018
(51) Int. Cl.: C12Q 1/04, B01L 3/00, G01N 21/3563, G01N 21/31

(54) **MIKROBIELLER TESTSTANDARD FÜR DIE VERWENDUNG IN DER INFRAROT-SPEKTROMETRIE**
MICROBIAL TEST STANDARD FOR USE IN INFRARED SPECTROMETRY
STANDARD D'ESSAI MICROBIENNE POUR UNE UTILISATION DANS UN SPECTROMÉTRIE INFRAROUGE

(30) Priorität: 19.04.2017 DE 102017108278
(43) Veröffentlichungstag der Anmeldung: 24.10.2018
(73) Patentinhaber: Bruker Daltonik GmbH, 28359 Bremen (DE)
(72) Erfinder: Maier, Thomas, 28865 Lilienthal Niedersachsen (DE); Mauder, Norman, 28211 Bremen (DE)
(74) Vertreter: Boßmeyer, Jens

(56) Entgegenhaltungen:
- WO-A2-2008/122975
- PEIREN JINDRICH ET AL: "Impact of the freeze-drying process on product appearance, residual moisture content, viability, and batch uniformity of freeze-dried bacterial cultures safeguarded at culture collections", APPLIED MICROBIOLOGY AND BIOTECHNOLOGY, SPRINGER, DE, Bd. 100, Nr. 14, 15. Februar 2016 (2016-02-15), Seiten 6239-6249, XP035986557, ISSN: 0175-7598, DOI: 10.1007/S00253-016-7359-1 [gefunden am 2016-02-15]

## Beschreibung

### Gebiet der Erfindung

Die Erfindung betrifft mikrobielle (insbesondere bakterielle) Teststandards für die Verwendung in der Infrarot-Spektrometrie, speziell in der Fourier-Transformations-Infrarot-Spektrometrie (FTIR), vorzugsweise in Transmission, als Hilfe und Prozesskontrolle bei Messungen zur Differenzierung, Identifizierung und/oder Charakterisierung von Mikroorganismen.

### Hintergrund der Erfindung

Der Stand der Technik wird im Folgenden mit Bezug auf einen speziellen Aspekt erläutert. Dies soll jedoch nicht als Einschränkung verstanden werden. Nützliche Fortentwicklungen und Änderungen vom aus dem Stand der Technik bekannten können auch über den vergleichsweise engen Rahmen dieser Einleitung hinaus anwendbar sein und werden sich geübten Praktikern auf diesem Gebiet nach der Lektüre der nachfolgenden Offenbarung umstandslos erschließen.

IR-Spektren, die für die mikrobielle Identifizierung bevorzugt in Transmission aufgenommen werden, beinhalten typischerweise eine Reihe von Extinktionssignalen oder Absorptionsbanden entlang eines vorgegebenen Bereichs von Wellenlängen, speziell im mittleren Infrarotbereich (etwa 4000 - 400 cm⁻¹). Mikroorganismen als zu untersuchende Proben hinterlassen deutliche und erkennbare spektrale Signaturen in diesen Spektren. Diese Signaturen weisen eine Überlagerung von Beiträgen aller Zellbestandteile, d.h. zytoplasmatischen Komponenten, Membran- und Zellwandkomponenten (also insbesondere Polysaccharide, DNS/RNS, Proteine), auf und geben damit die phänotypische Expression des Genoms (DNS/RNS) bzw. den genetischen Fingerabdruck wieder. Dieser Fingerabdruck-Charakter ist die Grundlage für die Verwendung der Infrarot-Spektrometrie bei der Differenzierung, Identifizierung und Charakterisierung von Mikroorganismen. Für Einzelheiten dieser Messmethoden sei auf die Übersicht von Dieter Naumann "Infrared Spectroscopy in Microbiology" in der Encyclopedia of Analytical Chemistry (R.A. Meyers (Ed.), Seiten 102-131, John Wiley & Sons Ltd, Chichester, 2000) verwiesen.

Heutzutage ist es möglich, unvorhergesehene und unerwünschte Schwankungen und Änderungen instrumenteller Einstellungen und Eigenschaften eines Infrarot-Spektrometers über eine bestimmte Betriebsdauer, die die Reproduzierbarkeit und Vergleichbarkeit von Messungen beeinflussen können, mittels automatisierter Prüf- und Rekalibrierungsroutinen einigermaßen verlässlich zu erkennen und auszugleichen.

Allerdings können die äußeren Messbedingungen über einen Betriebszeitraum, ggfs. sogar von Aufnahme zu Aufnahme, variieren. Häufig sind Infrarot-Spektrometer nicht mit konditionierten Probenträgerschächten ausgestattet, die für gleichbleibende und beständige Umgebungsbedingungen wie Druck, Temperatur und Feuchte während der und zwischen den einzelnen Aufnahmen sorgen. Es erscheint plausibel, dass insbesondere ein veränderter Feuchtegehalt die spektralen Eigenschaften einer präparierten Probe zum Beispiel durch Bildung von Hydrathüllen oder Kristallwasser beeinflussen und damit die Vergleichbarkeit ähnlicher Proben oder sogar identischer Replikate beeinträchtigen kann. Diese Unterschiede würden dann insbesondere im Wechsel von Winter (kalte Luft, geringe absolute Feuchte) und Sommer (warme Luft, hohe absolute Feuchte) hervortreten. Es besteht daher ein Bedarf nach verbesserter Prozessüberwachung in der Infrarot-Spektrometrie, um diesen etwaigen äußeren Einflüssen besser Rechnung zu tragen und IR-Messungen zeitnah bewerten zu können.

Prozesskontrollen in einfacher Form sind bei IR-Messungen bereits bekannt. In der internationalen Anmeldung WO 99/16895 A1 beispielsweise werden zur Überwachung der spektralen Reproduzierbarkeit vier Chargen der zu identifizierenden Mikroorganismen jeweils dreifach gemessen und dann die Ähnlichkeit der jeweiligen Replikate zueinander bewertet.

Eine wesentliche Aufgabe der Differenzierung, Identifizierung oder Charakterisierung von Mikroorganismen mittels IR-Spektrometrie ist es, Stämme zu differenzieren und ihre phänotypischen Beziehungen zueinander zu bewerten. Die spezifischen Absorptionsspektren mikrobieller Biomasse werden aufgenommen und verglichen, um die spektralen Abstände mit Hilfe leistungsfähiger Clusteranalyse-Algorithmen zu ermitteln und zu veranschaulichen, damit die Verwandtschaftsbeziehungen für den Benutzer erkennbar werden. Folgende Herausforderungen stellen sich dabei: einerseits ist eine hohe spektrale Unterscheidungskraft erforderlich, um insbesondere zwei verschiedene Stämme derselben mikrobiellen Art (Spezies) auseinander halten zu können; andererseits müssen identische Stämme, selbst wenn sie von unterschiedlichen Sammelpunkten stammen, bei der Analyse zuverlässig in denselben Cluster fallen, also in den auf den Probenträger aufgetragenen Replikaten möglichst geringe intramikrobielle spektrale Abstände zueinander und untereinander aufweisen, um eine verlässliche Reproduzierbarkeit der Messungen auszuweisen.

Die internationale Anmeldung WO 2008/122975 A2 offenbart ein Verfahren zum Detektieren und/oder Identifizieren spezifischer Bakterien in einer unkultivierten Probe. Das Verfahren umfasst Schritte, die unter anderem ausgewählt sind aus (a) Erhalten eines Absorptionsspektrums (AS) der unkultivierten Probe; (b) Erfassen der n-dimensionalen Volumengrenzen für die spezifischen Bakterien; (c) Datenverarbeitung des AS; und (d) Detektieren und/oder Identifizieren der spezifischen Bakterien, wenn die statistische m1-Korrelation und/oder die m Merkmale innerhalb des n-dimensionalen Volumens liegen.

Es besteht daher ein Bedarf, mikrobielle Teststandards sowie geeignete Verfahren zu deren Verwendung zur Verfügung zu stellen, mit denen in Kenntnis der Identität und Eigenschaften der gemessenen Referenzbiomasse die Reproduzierbarkeit und spektrale Unterscheidungskraft der IR-spektrometrischen Messung sowie der nachfolgenden Auswerteschritte bestimmt und beurteilt werden können.

### Kurze Beschreibung der Erfindung

Gemäß einem ersten Aspekt betrifft die Erfindung einen mikrobiellen Teststandard für die Verwendung in der Infrarot-Spektrometrie, der wenigstens zwei wiederverschließbare, im geschlossenen Zustand flüssigkeitsdichte Gefäße aufweist, von denen jedes eine vorbestimmte Menge getrockneter Biomasse eines Mikroorganismus enthält, wobei sich die Mikroorganismen in den verschiedenen Gefäßen in wenigstens einer Eigenschaft unterscheiden, welche insbesondere aus der Gruppe Art, Unterart, Stamm, Serovar, Pathovar, Toxivar und Varietät gewählt werden kann, und sich der Unterschied in einem vorbestimmten intermikrobiellen spektralen Abstand offenbart, dadurch gekennzeichnet, dass die getrocknete Biomasse sterilisiert ist.

Der Einsatz des Teststandards erfolgt vorzugsweise folgendermaßen: zusammen mit den eigentlichen Proben unbekannter Mikroorganismen werden (wenigstens) zwei Referenz-Mikroorganismen, die einen vorbestimmten intermikrobiellen spektralen Abstand zueinander aufweisen, in Duplikaten oder auch einer höheren Anzahl an Replikaten räumlich getrennt auf die gleiche Probenträgerplatte aufgetragen. Die Referenzbiomassen werden vor den "echten" zu identifizierenden Proben IR-spektrometrisch gemessen.

Um den Test zu bestehen und die Messung der Probenträgerplatte insgesamt als gültig auszuweisen, müssen die intermikrobiellen spektralen Abstände zwischen den beiden Referenz-Mikroorganismen größer als ein spektraler Mindestabstand B sein, um eine ausreichende spektrale Unterscheidungskraft nachzuweisen. Bei Auftrag in Duplikaten können vier intermikrobielle spektrale Abstandswerte berechnet werden, von denen jeder der Bedingung zu genügen hat, um den Test zu bestehen. Werden mehr als zwei Replikate pro Mikroorganismus aufgetragen, zum Beispiel fünf bis zehn, so kann alternativ zu einer 100%igen Bedingungserfüllung ein bestimmtes Quantil von intermikrobiellen spektralen Abständen, beispielsweise zwei bis zehn Prozent, bevorzugt fünf Prozent, die Grenzbedingungen nicht einhalten, ohne zu einem Nichtbestehen des Tests zu führen. Zum Beispiel ergeben 5 × 5 Probenflecken 25 intermikrobielle spektrale Abstände zwischen den Replikaten der beiden Referenzbiomassen; eine geringe Zahl davon, beispielsweise einer dieser Abstandswerte kleiner als B könnte tolerierbar sein, ohne die Messung insgesamt als ungültig auszuweisen.

Ähnliche Überlegungen gelten in Varianten dieses Verfahrens natürlich auch für den intramikrobiellen spektralen Höchstabstand A bei entsprechend hoher Zahl an aufgetragenen Replikaten. Um ein Mindestmaß an spektraler Reproduzierbarkeit nachzuweisen, sollten die intramikrobiellen spektralen Abstände der Replikate zueinander und untereinander, oder zumindest ein großes Quantil davon (>90%), geringer als dieser bestimmte Höchstabstand A sein.

Anstatt eine IR-Messung mit einer (Warn-) Kennzeichnung für eine fehlgeschlagene Messung zu versehen, kann ein geübter Praktiker die (teilweise) Nichterfüllung der Testkriterien auch nutzen, um sich die IR-Spektren im Detail anzuschauen und daraus Schlussfolgerungen zu ziehen, die es ermöglichen, die Messungen bei veränderten und optimierten Bedingungen zu wiederholen, sofern möglich. Zum einen kann ein optimierter Feuchtegehalt im Probenträgerschacht zu besseren Ergebnissen führen. Wenn das Signal-zu-Rausch-Verhältnis der Absorptionsbanden zu niedrig ist, können mehrere Aufnahmen des gleichen Probenflecks aufaddiert werden, um die Datengrundlage für die Auswertung zu verbessern. Schließlich kann auch die Präparation der Mikroorganismen-Proben auf dem Probenträger dahingehend geprüft werden, ob sie mit weitgehend gleichförmiger Schichtdicke aufgetragen wurden. Falls sich das nicht bestätigt, lässt sich die Präparation mit besonderem Augenmerk auf diese Eigenschaft erneut ausführen; und diese Optimierungsversuche können mit dem mikrobiellen Teststandard begleitet und geprüft werden.

Neben den offensichtlichen taxonomischen Hierarchieebenen Art (Spezies) und Unterart (Subspezies) können verschiedene detailliertere Eigenschaften von Mikroorganismen einen intermikrobiellen spektralen Abstand ausmachen. Der Begriff "Stamm" (genauer "Kulturstamm" oder "Abstammungslinie", englisch *strain*) zum Beispiel beschreibt eine Population, die aus einem einzelnen Organismus heraus vermehrt wurde und in einer (häufig staatlichen) Sammelstelle für Mikroorganismenstämme aufbewahrt wird, wobei an die Namenskette mit Gattung, Art, Unterart und Varietätentyp eine international normierte Stammbezeichnung angefügt wird. Die einzelnen Organismen eines Stammes sind genetisch identisch; verschiedene Stämme variieren dagegen leicht in ihrer genetischen Ausstattung und bieten daher eine Vielzahl an möglichen Stamm-Kombinationen mit unterschiedlichen spektralen Eigenschaften und Abständen, die für die Verwendung als mikrobieller Teststandard in der IR-Spektrometrie geeignet sein können.

Andere spektrale Unterscheidungsmerkmale können sich beispielsweise in Serotypen oder Serovaren äußern. Mit Serotyp oder Serovar (Kurzform von *Serovarietas*) bezeichnet man Varietäten innerhalb von Subspezies von Bakterien, die mit serologischen Tests unterscheidbar sind: sie unterscheiden sich durch Antigene an der Oberfläche der Zellen und werden in der klassischen Mikrobiologie mittels spezifischer Antikörper identifiziert. Die taxonomische Hierarchie für Serotypen ist die folgende: Gattung > Art (Spezies) > Unterart (Subspezies, subsp.) > Serotyp, beispielsweise mit dem ergänzten binomischen Artnamen *Salmonella enterica* subsp. *enterica* serotyp Typhi, Kurzform *Salmonella* Typhi.

Ein Pathovar (von griechisch pathos "Leiden") ist ein Bakterienstamm oder eine Gruppe von Stämmen mit denselben Eigenschaften, die innerhalb der Art oder Unterart aufgrund ihrer Pathogenität von anderen Stämmen abgegrenzt werden können. Pathovare werden mit Dritt- oder Viertzusatz zum binomischen Artnamen gekennzeichnet. Das Bakterium *Xanthomonas axonopodis* beispielsweise, das Zitronenkrebs verursachen kann, hat verschiedene Pathovare mit unterschiedlichen Wirtsspezialisierungen: *X. axonopodis* pv. *citri* ist eins davon, die Abkürzung "pv." bedeutet "Pathovar". Auch die virulenten Stämme humanpathogener Erreger besitzen Pathovare, die hier aber durch Namensvorsätze gekennzeichnet werden. Das zumeist völlig harmlose Darmbakterium *Escherichia coli* weist zum Beispiel die höchst gefährlichen Pathovare *enterohämorrhagische E. coli* (EHEC), *enteropathogene E. coli* (EPEC), *enterotoxinbildende E. coli* (ETEC), *enteroinvasive E. coli* (EIEC), *enteroaggregative E. coli* (EAEC) sowie *diffus adhärente E. coli* (DAEC) auf. Die Pathovare können wiederum verschiedene Serotypen umfassen: so gibt es bei EHEC viele bekannte Serotypen, wobei etwa 60 Prozent aller identifizierten EHEC-Serotypen auf O157, O103, und O26 entfallen. Besonders gefährlich ist der Sero-Untertypus O157/H7. Es versteht sich, dass für mikrobielle Teststandards im alltäglichen Laborgebrauch für den Menschen harmlose Mikroorganismen deutlich zu bevorzugen sind.

Im weiteren Sinne können Mikroorganismen auch in Varietäten eingeteilt werden, die sich durch andere medizinisch relevante Merkmale unterscheiden, insbesondere durch das Resistenzverhalten gegenüber Antibiotika (insbesondere Beta-Lactam-Antibiotika und Glykopeptid-Antibiotika), aber auch durch die Toxinbildung ("Toxivaren") oder durch die Empfänglichkeit für gleiche oder ähnliche Bakteriophagen ("Phagovaren"). Ganz allgemein spricht man von "Biovaren", wenn eine Auswahl von Mikroben einer Spezies oder Subspezies biologische Gemeinsamkeiten besitzt. Ein Beispiel für eine antibiotika-resistente Varietät ist MRSA: *Methicillin-resistenter Staphylococcus aureus.*

Die getrocknete Biomasse ist sterilisiert, um messverfälschenden mikrobiellen Kontaminationen vorzubeugen; insbesondere kann sie vakuumgetrocknete Pellets aufweisen. Diese Bereitstellungsform eignet sich besonders für die schnelle Aufbereitung einer für die Erzeugung von Replikaten auf dem Probenträger dienenden Teststandard-Suspension im Labor, wenn IR-Messungen zeitnah durchgeführt werden sollen.

In einer bevorzugten Ausführungsform gehören die Mikroorganismen verschiedenen Stämmen einer Bakterienart an. Als Beispiel sei die Art *Escherichia coli* genannt, wobei die getrocknete Biomasse in den verschiedenen Gefäßen in einer besonders bevorzugten Ausführung die Stämme DH5α (DSM 6897) und ML3 (DSM 1058) umfassen kann. Zwei Bakterienstämme erlauben die Bestimmung eines definierten intermikrobiellen spektralen Abstands, um auf diese Weise die Reproduzierbarkeit zwischen den Replikaten eines Stammes und die spektrale Unterscheidungskraft zwischen den einzelnen Messungen der verschiedenen Stämme zu überwachen und bewerten. Es ist besonders vorteilhaft, wenn die Stämme regelmäßig in mikrobiologischen Proben auftreten und daher eine hinreichende Praxisnähe aufweisen; bei *E*. *coli*-Stämmen ist das der Fall. Der intermikrobielle spektrale Abstand kann sich in einem Abstand zweier Hauptkomponenten einer Hauptkomponentenanalyse äußern. In weiteren bevorzugten Ausführungsformen kann der spektrale Abstand aber auch euklidisch sein, was der Differenz der n-dimensionalen spektralen Vektoren entspricht. Die Dimensionalität kann je nach Auflösung oder gewähltem Wellenzahlbereich im zweistelligen Bereich anfangen und bis zu mehrere hundert oder darüber hinaus reichen, beispielsweise 20 < n < 2000; insbesondere 400 < n < 600; zum Beispiel n = 500.

Die Gefäße können Schraubverschlussdeckel zum sicheren Öffnen und Wiederverschließen umfassen. Auch nach Einfüllen eines Lösungsmittels in die Gefäße zum Aufbereiten einer Mikroorganismensuspension lässt sich letztere über einen längeren Zeitraum (z.B. bis zu zehn Wochen) zur weiteren Verwendung aufbewahren, ohne dass die spektrale Aussagekraft beeinträchtig wird.

Gemäß einem zweiten Aspekt betrifft die Erfindung eine Verwendung eines solchen mikrobiellen Teststandards, aufweisend: - Bereitstellen eines Probenträgers für die Infrarot-Spektrometrie, der ein Feld aus Probenflecken aufweist, typischerweise 48, 96 oder 384 in Matrix-Reihen-Spalten-Anordnung; - Resuspendieren der Biomasse in den Gefäßen durch Einfüllen eines Lösungsmittels, beispielsweise destilliertes und/oder deionisiertes Wasser; - Entnehmen einer vorbestimmten Menge der Suspension aus jedem Gefäß und deren Aufbringen auf eine vorbestimmte Anzahl von Probenflecken, bevorzugt in Duplikaten oder Triplikaten; - Eintrocknen der Suspension auf den Probenflecken, gegebenenfalls unter Einwirkung von Wärme und Konvektion; - Aufnehmen von Infrarot-Spektren von den Probenflecken, vorzugsweise in Transmission; - Errechnen intermikrobieller spektraler Abstände zwischen spektralen Signaturen in den Infrarot-Spektren von den verschiedenen Probenflecken; und - Abfragen, ob die spektralen Abstände außerhalb eines vorbestimmten Bereiches liegen, wobei die IR-Spektren von dem Probenträger insgesamt mit einem entsprechenden (Warn-) Hinweis gekennzeichnet werden können, wenn dies der Fall ist.

In verschiedenen Ausführungsformen können die spektralen Abstände in einem vorbestimmten Wellenzahlbereich, vorzugsweise zwischen etwa 1300 und 800 cm⁻¹ zur Verringerung des Einflusses von störenden Wasserabsorptionsbanden, aus den mikrobiellen spektralen Signaturen errechnet werden und einem vorbestimmten Abstand entsprechen, beispielsweise direkt den euklidischen Abständen der spektralen Vektoren. Eine Analyse einer größeren Anzahl von Spektren könnte hier auch mit Hilfe einer hierarchischen Cluster-Analyse (HCA) erfolgen. Oder aber die Berechnung erfolgt an Hand einer Hauptkomponentenanalyse der mikrobiellen spektralen Signaturen, und die Abstände entsprechen dann einem vorbestimmten Abstand im Hauptkomponentenraum. Alternative Verfahren zur Unterscheidung verschiedener Mikroorganismen aus dem Bereich des überwachten Maschinenlernens sind beispielsweise ANN (*artificial neural network analysis;* künstliche neuronale Netzwerk-Analyse), PLS-DA (*partial least-square discriminant analysis;* Diskriminanzanalyse mit partiellen kleinsten Quadraten) sowie SVM (*support vector machines;* Stützvektormethode). In diesen Fällen sind jedoch nicht in erster Linie die spektralen Abstände entscheidend sondern die korrekte Zuordnung der Testspektren zu den erwarteten Klassen.

Zusätzlich zu den intermikrobiellen Abständen können intramikrobielle spektrale Abstände (also Abstände zwischen Replikaten des gleichen Mikroorganismus) zwischen den spektralen Signaturen in den Infrarot-Spektren von den verschiedenen Probenflecken zwecks Prüfung der spektralen Reproduzierbarkeit errechnet werden.

Vorzugsweise erstreckt sich der vorbestimmte Bereich (i) des intramikrobiellen spektralen Abstands bis zu einem Höchstabstand A, der ein Maß für eine Reproduzierbarkeit der Aufnahmen ist, und (ii) des intermikrobiellen spektralen Abstands von einem Mindestabstand B, der ein Maß für eine spektrale Unterscheidungskraft in den Aufnahmen ist. In verschiedenen Ausführungsformen kann eine geringe Zahl (z.B. zwei bis zehn Prozent, insbesondere fünf Prozent) von spektralen Abständen zwischen den IR-spektrometrischen Aufnahmen der verschiedenen vereinzelten Probenflecken die Abstandsbedingungen nicht erfüllen, ohne dass ein solcher Befund zur negativen Kennzeichnung oder sogar Ungültigerklärung aller Messungen von diesem Probenträger führt, sofern eine bedeutende Mehrheit der Abstände es tut.

Durch Agitieren der Gefäße lässt sich das Resuspendieren im Sinne einer gleichmäßigen Verteilung der suspendierten Mikroorganismenzellen unterstützen, wobei insbesondere Gefäße mit Schraubverschlüssen eine verlässliche Absicherung der Umgebung gegenüber unerwünschtem Austritt von mikrobiellen Aerosolen gewährleisten sowie für eine wochenlange Lagerung verbleibender Suspension zur späteren Verwendung geeignet sind. Gegebenenfalls kann das Resuspendieren zusätzlich oder alternativ auch durch Mischpipettieren bei geöffnetem Gefäß unterstützt werden.

In verschiedenen Ausführungsformen kann eine Haltbarkeit der Suspensionen durch Zugabe von Ethanol verlängert werden. Nach einer solchen Maßnahme lassen sich die angerichteten Suspensionen wochenlang, insbesondere bis zu zehn Wochen, für späteren Gebrauch in der Messungsüberwachung und Prozesskontrolle lagern.

Besonders praktikabel ist es, die Infrarot-Spektren unter Verwendung einer FourierTransformation auszuwerten, da sich so unbekannte wiederkehrende Extinktionssignale mit hoher Empfindlichkeit nachweisen lassen.

### Kurze Beschreibung der Abbildungen

Zum besseren Verständnis der Erfindung wird auf die folgenden Abbildungen verwiesen. Die Elemente in den Abbildungen sind nicht unbedingt maßstabsgetreu dargestellt, sondern sollen in erster Linie die Prinzipien der Erfindung (größtenteils schematisch) veranschaulichen. In den Abbildungen sind einander entsprechende Elemente in den verschiedenen Ansichten durch gleiche Bezugszeichen gekennzeichnet.
Abbildung 1A zeigt schematisch den ersten Teil einer beispielhaften Verwendung eines mikrobiellen Teststandards.
Abbildung 1B veranschaulicht schematisch eine IR-spektrometrische Messung in Transmission durch getrocknete Proben mit dazugehörigem Beispiel eines Extinktionsspektrums.
Abbildung 1C erläutert schematisch verschiedene Bearbeitungsschritte eines Extinktionsspektrums, die vor der Auswertung vorgenommen werden.
Abbildung 2 illustriert schematisch das mögliche Ergebnis einer spektralen Abstandsbestimmung für Replikate zweier Bakterienstämme in einem zweidimensionalen Hauptkomponentenraum.

### Detaillierte Beschreibung

Während die Erfindung anhand einer Anzahl von Ausführungsformen dargestellt und erläutert wurde, werden Fachleute auf dem Gebiet anerkennen, dass verschiedene Änderungen in Form und Detail daran vorgenommen werden können, ohne vom Umfang der in den beigefügten Patentansprüchen definierten technischen Lehre abzuweichen.

Die Herstellung der getrockneten Biomasse für den mikrobiellen IR-Teststandard kann folgendermaßen erfolgen: Die verschiedenen Referenz-Mikroorganismen werden auf einem flächigen Nährmedium (z.B. Columbia-Schafblut-Agar) über Nacht gezüchtet. Die gezüchteten Zellen werden in einige hundert Milliliter Nährbouillon (z.B. LB - englisch *lysogeny broth*) eingeimpft, um sie dann bei 37°C unter leichter Agitation von wenigen hundert *rpm* für einige Stunden (z.B. 12 bis 24h) nachwachsen zu lassen. Die derart angereicherte Bouillon kann dann auf verschiedene Zentrifugiergefäße aufgeteilt und bei einigen tausend g für einige Minuten abzentrifugiert werden. Der Überstand wird entsorgt, und das verbleibende mikrobielle Pellet wird zwecks Entfernung von Nährbouillonresten in Wasser resuspendiert. Erneute Zentrifugation und erneutes Resuspendieren in Wasser, ggfs. ergänzt durch Zugabe eines protischen Lösungsmittels wie Ethanol, ergeben eine Suspension, deren Mikroorganismengehalt über eine Messung der optischen Dichte (z.B. gemäß McFarland-Standard) bestimmt werden kann. Ist die Konzentration der suspendierten Mikroorganismen ausreichend, kann die Suspension auf Kunststoffgefäße aliquotiert und darin eingetrocknet werden, zum Beispiel im Vakuum bei leicht erhöhten Temperaturen, um ein von Flüssigkeit befreites, gebrauchsfertiges Pellet direkt im Gefäß zu bilden.

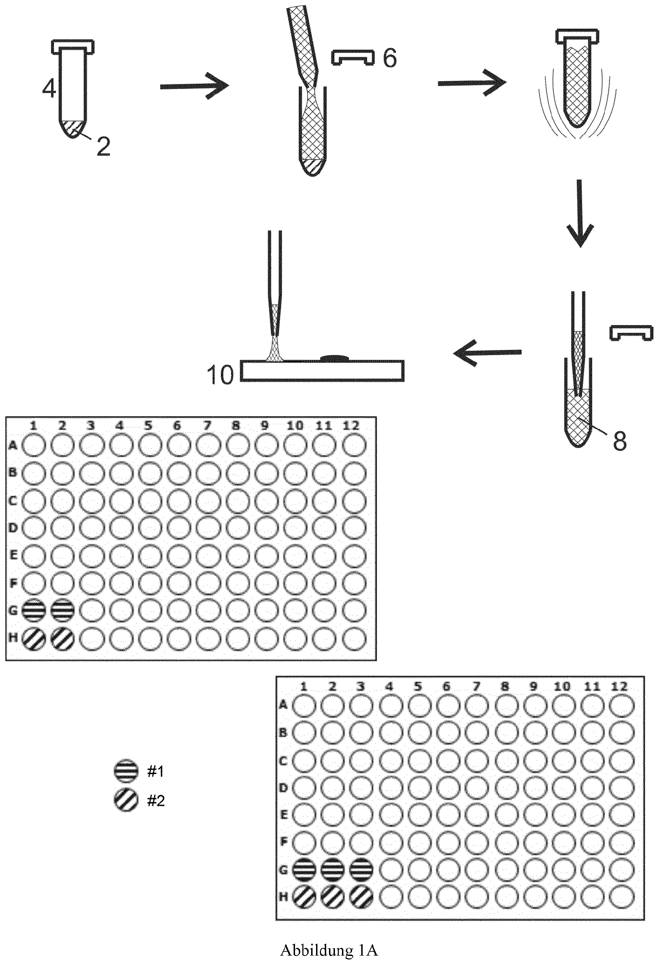

Die Abbildungen 1A bis 1C skizzieren einen möglichen Ablauf für die Verwendung eines mikrobiellen Teststandards.

Die getrocknete Biomasse (2) für die beiden Referenz-Mikroorganismen kann in Kunststoffbehältnissen (4) mit Schraubverschlussdeckel (6) bereitgestellt werden. Zur Vorbereitung einer Referenzprobe wird der Schraubverschlussdeckel (6) abgenommen und eine Menge an Lösungsmittel wie destilliertem oder deionisiertem Wasser eingefüllt, um die trockenen Pellets (2) zu resuspendieren. Dieser Vorgang kann, nach dem Wiederverschließen der Gefäße (4), durch leichtes Schütteln oder, zusätzlich oder alternativ, durch wiederholtes Einziehen in und Herausdrücken aus einer Pipettenspitze ("Mischpipettieren") ohne Blasenbildung unterstützt werden (nicht gezeigt).

Nach einigen Minuten, wenn sich die feste Biomasse "aufgelöst" hat und nicht mehr sichtbar ist, lassen sich die Deckel (6) wieder abnehmen und eine Menge der mikrobiellen Suspension (8) den Gefäßen (4) entnehmen und auf eine Anzahl von Probenflecken (A-H; 1-12) auf einem IR-Spektrometrie-Probenträger (10) auftragen. Dies kann in Duplikaten, Triplikaten oder einer höheren Anzahl an Replikaten erfolgen, wie für zwei Probenträger (10) mit 96-Probenfleck-Anordnung (acht Reihen A-H, zwölf Spalten 1-12) schematisch gezeigt. Grundsätzlich gilt, dass mit zunehmender Zahl der Replikate des Teststandards die statistische Grundlage der spektralen Abstandsbestimmung verbessert werden kann; allerdings auf Kosten einer entsprechend geringeren Zahl von Probenflecken für die eigentlich zu identifizierenden analytischen Proben auf dem Probenträger, die hier der Übersichtlichkeit halber nicht dargestellt sind. Die Tropfen der Teststandard-Suspension werden getrocknet, ggfs. unterstützt durch Einstrahlung von Wärme bei gegenüber Raumtemperatur mäßig erhöhter Temperatur und/oder einen Luftstrom.

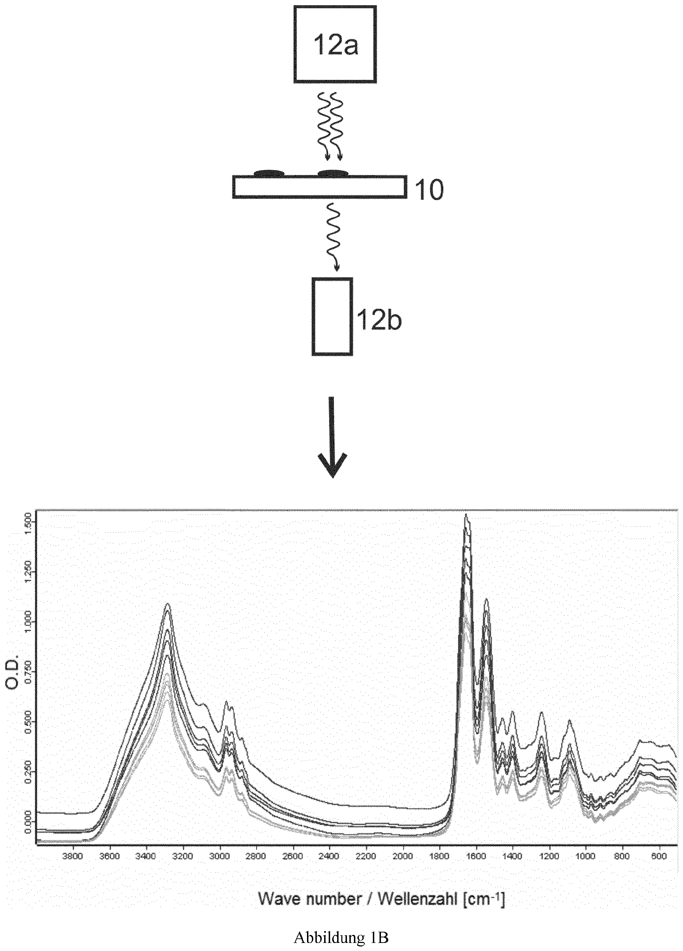

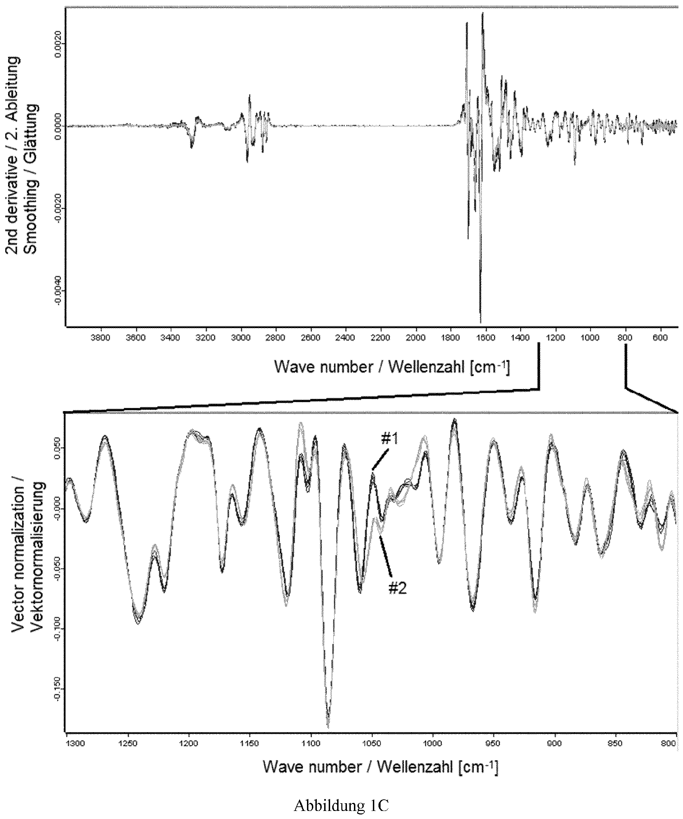

Die Mikroorganismen des Referenzstandards (#1, #2) werden dann im gleichen Durchgang wie die eigentliche Probe mit einem IR-Spektrometer (12a, 12b) gemessen, vorzugsweise in Transmission wie gezeigt. Ein Beispiel für ein solches Spektrometer ist das TENSOR II FT-IR von Bruker Optik GmbH. Das Ergebnis einer solchen Messung ist ein Extinktionsspektrum, wie es beispielhaft unten in der Abbildung 1B dargestellt ist. Aufgetragen ist die optische Dichte (O.D.) gegen die Wellenzahl wie in der Spektrometrie üblich. Die aufgenommen Spektren werden zweifach abgeleitet und jeweils über eine bestimmte Anzahl von Datenpunkten geglättet. Anschließend wird ein Wellenzahlbereich ausgewählt, in dem die Absorptionsbanden mikrobieller Zellen, die insbesondere durch Kohlenhydrate und Proteine verursacht werden, am besten gegenüber Hintergrundeinflüssen wie Wasserabsorptionsbanden hervortreten und daher das beste Signal-zu-Untergrund-Verhältnis liefern. Der Bereich zwischen etwa 1300 und 800 cm⁻¹ ist hierfür besonders geeignet; Abbildung 1C.

Eine Vektornormalisierung bereitet den ausgewählten Spektralbereich auf die anschließende Auswertung hinsichtlich spektraler Abstandsmaße vor. Eine Möglichkeit, den spektralen Abstand aus den Mikroorganismus-spezifischen Extinktionssignalen zu bestimmen, besteht in einer Hauptkomponenten-Analyse. Entscheidend ist dabei, dass die Mikroorganismen für die verschiedenen Gefäße so gewählt sind, dass sie sich verlässlich, knapp aber deutlich in unterschiedlichen Komponenten darstellen lassen. Der intermikrobielle spektrale Abstand ist zwar wesentlich durch eine Untergrenze definiert, sollte aber trotzdem nicht zu groß gewählt sein, um eine Aussage über die spektrale Unterscheidungskraft an der Leistungsgrenze des Infrarot-Spektrometers zu erlauben. Er sollte auch nicht zu klein sein, um der Gefahr einer streuungsbedingten, zufälligen Überlappung der Hauptkomponenten-Datenwolken, welche von Messung zu Messung eine gewisse Varianz zeigen, vorzubeugen.

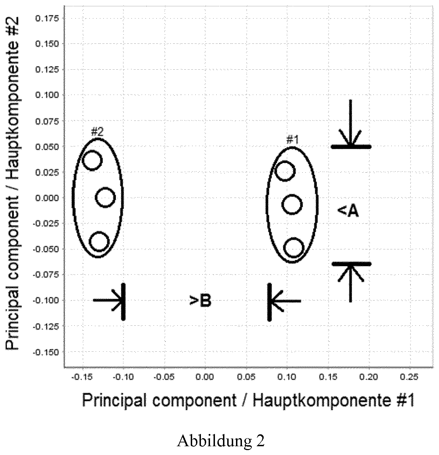

Abbildung 2 zeigt beispielhaft und schematisch ein Ergebnis einer Hauptkomponentenanalyse auf der Grundlage von IR-Spektren zweier Bakterienstämme. Der intramikrobielle spektrale Abstand zwischen den Replikaten des gleichen Mikroorganismus darf einen bestimmten Höchstwert A nicht übersteigen, weil ansonsten die Identität nicht bestätigt werden könnte, was die Güte der Messung in Zweifel ziehen würde. Gleichfalls darf der intermikrobielle spektrale Abstand zwischen den verschiedenen Replikaten der verschiedenen eingesetzten Mikroorganismen eine bestimmte Untergrenze B nicht unterschreiten, da ansonsten die spektrale Unterscheidungskraft der Messung nicht ausreichend gewesen ist. Je höher die Zahl der Replikate der einzelnen Mikroorganismen-Suspensionen ist, desto statistisch zuverlässiger werden die daraus ableitbaren Aussagen, wobei jedoch auch der vorhandene Platz für die eigentlichen mikrobiellen Messproben auf dem Probenträger zu berücksichtigen ist.

Wie in Abbildung 2 zu sehen ist, bilden sich bei wiederholter Messung der gleichen Mikroorganismen zwei angedeutete vereinzelte Datenwolken im zweidimensionalen Hauptkomponentenraum aus, die bezüglich der einzelnen Replikate eines Organismus eng beieinander liegen, von den Replikaten des jeweils anderen Organismus jedoch deutlich entfernt sind (in dem gezeigten Beispiel > 0,15 willkürliche Einheiten). Da der mikrobielle Teststandard im gleichen Messvorgang untersucht wird wie die echten mikrobiellen Proben, erlaubt dieser Abstand in den errechneten Hauptkomponenten eine Aussage über die spektrale Unterscheidungskraft des IR-Spektrometers. Gleichzeitig kann durch die intramikrobiellen spektralen Abstände der einzelnen Replikate des gleichen Mikroorganismus zu- und untereinander die Reproduzierbarkeit der IR-Messung bewertet werden. Sollten einzelne Datenpunkte aus den Replikaten des gleichen Organismus den zulässigen Höchstabstand A überschreiten, wäre dies als Anzeichen eines Problems der Reproduzierbarkeit der Messungen zu werten. Die parallel ausgeführten Messungen realer Proben lassen sich dann mit einer entsprechenden Kennzeichnung versehen. Sollte es sogar vorkommen, dass die Datenwolken der verschiedenen Mikroorganismen ineinander übergehen, wäre die spektrale Unterscheidungskraft nicht gewährleistet, was die Güte der Identifizierung verringert, wenn nicht sogar das Identifizierungs- oder Charakterisierungsergebnis von dem nämlichen Probenträger unter den gegebenen Messbedingungen als unzuverlässig zu kennzeichnen ist.

Der Einsatz einer Hauptkomponentenanalyse ist vorstehend nicht als beschränkend sondern als veranschaulichendes Beispiel zu verstehen. Prinzipien der vorliegenden Offenbarung lassen sich auch mit alternativen Verfahren zur Bestimmung spektraler Abstände ausführen, beispielsweise durch die nativen euklidischen Distanzen und/oder unter Zuhilfenahme hierarchischer Cluster-Analysen. Auch wäre eine Klassifizierung der Testspektren mit zuvor entsprechend trainierten ANN (*artificial neural network analysis*), PLS-DA (*partial least-square discriminant analysis*) oder SVM (*support vector machines*) möglich.

Vorstehend sind die Prinzipien der Erfindung an Hand zweier spektral voneinander unterscheidbarer Mikroorganismen erläutert worden. Fachleute werden jedoch erkennen, dass auch mehr als zwei geeignete Mikroorganismen zur Bereitstellung eines mikrobiellen Teststandards für die Infrarot-Spektrometrie verwendet werden können. Der Grundgedanke lässt sich diesbezüglich beliebig erweitern.

Die Erfindung ist vorstehend mit Bezug auf verschiedene besondere Ausführungsbeispiele beschrieben. Es versteht sich jedoch, dass diverse Aspekte oder Details der beschriebenen Ausführungen geändert werden können, ohne vom Umfang der Erfindung abzuweichen. Insbesondere können im Zusammenhang mit unterschiedlichen Ausführungsformen offenbarte Merkmale und Maßnahmen beliebig kombiniert werden, sofern dies einem Fachmann praktikabel erscheint. Überdies dient die vorstehende Beschreibung nur zur Veranschaulichung der Erfindung und nicht zur Einschränkung des Schutzbereichs, der ausschließlich durch die beigefügten Ansprüche definiert wird.

## Patentansprüche

1. Mikrobieller Teststandard für die Verwendung in der Infrarot-Spektrometrie, der wenigstens zwei wiederverschließbare, im geschlossenen Zustand flüssigkeitsdichte Gefäße aufweist, von denen jedes eine vorbestimmte Menge getrockneter Biomasse eines Mikroorganismus enthält, wobei sich die Mikroorganismen in den verschiedenen Gefäßen in wenigstens einer Eigenschaft unterscheiden und sich der Unterschied in einem vorbestimmten intermikrobiellen spektralen Abstand offenbart,
**dadurch gekennzeichnet, dass**
die getrocknete Biomasse sterilisiert ist.

2. Mikrobieller Teststandard nach Anspruch 1, **dadurch gekennzeichnet, dass** die wenigstens eine Eigenschaft aus der Gruppe Art, Unterart, Stamm, Serovar, Pathovar, Toxivar und Varietät gewählt ist.

3. Mikrobieller Teststandard nach Anspruch 1 oder 2, **dadurch gekennzeichnet, dass** die getrocknete Biomasse vakuumgetrocknete Pellets aufweist.

4. Mikrobieller Teststandard nach einem der Ansprüche 1 bis 3, **dadurch gekennzeichnet, dass** die Mikroorganismen verschiedenen Stämmen einer Bakterienart angehören.

5. Mikrobieller Teststandard nach einem der Ansprüche 1 bis 4, **dadurch gekennzeichnet, dass** die Gefäße Schraubverschlussdeckel aufweisen.

6. Verwendung des mikrobiellen Teststandards nach einem der Ansprüche 1 bis 5, aufweisend:
- Bereitstellen eines Probenträgers für die Infrarot-Spektrometrie, der ein Feld aus Probenflecken aufweist,
- Resuspendieren der Biomasse in den Gefäßen durch Einfüllen eines Lösungsmittels,
- Entnehmen einer vorbestimmten Menge der Suspension aus jedem Gefäß und deren Aufbringen auf eine vorbestimmte Anzahl von Probenflecken,
- Eintrocknen der Suspension auf den Probenflecken,
- Aufnehmen von Infrarot-Spektren von den Probenflecken,
- Errechnen intermikrobieller spektraler Abstände zwischen spektralen Signaturen in den Infrarot-Spektren von den verschiedenen Probenflecken, und
- Abfragen, ob die spektralen Abstände außerhalb eines vorbestimmten Bereiches liegen.

7. Verwendung nach Anspruch 6, **dadurch gekennzeichnet, dass** die spektralen Abstände aus den mikrobiellen spektralen Signaturen in einem vorbestimmten Wellenzahlbereich errechnet werden und einem vorbestimmten Abstand entsprechen.

8. Verwendung nach Anspruch 7, **dadurch gekennzeichnet, dass** der vorbestimmte Wellenzahlbereich zwischen etwa 1300 und 800 cm⁻¹ liegt.

9. Verwendung nach einem der Ansprüche 6 bis 8, **dadurch gekennzeichnet, dass** intramikrobielle spektrale Abstände zwischen den spektralen Signaturen in den Infrarot-Spektren von den verschiedenen Probenflecken zwecks Prüfung der spektralen Reproduzierbarkeit errechnet werden.

10. Verwendung nach Anspruch 9, **dadurch gekennzeichnet, dass** sich der vorbestimmte Bereich des intramikrobiellen spektralen Abstands bis zu einem Höchstabstand A, der ein Maß für eine Reproduzierbarkeit der Aufnahmen ist, und des intermikrobiellen spektralen Abstands von einem Mindestabstand B, der ein Maß für eine spektrale Unterscheidungskraft in den Aufnahmen ist, erstreckt.

11. Verwendung nach einem der Ansprüche 6 bis 10, **dadurch gekennzeichnet, dass** das Lösungsmittel zum Resuspendieren destilliertes und/oder deionisiertes Wasser umfasst.

12. Verwendung nach einem der Ansprüche 6 bis 11, **dadurch gekennzeichnet, dass** das Resuspendieren durch Agitieren der Gefäße oder Mischpipettieren unterstützt wird.

13. Verwendung nach einem der Ansprüche 6 bis 12, **dadurch gekennzeichnet, dass** den IR-Spektren ein (Warn-) Hinweis angefügt wird, wenn ein spektraler Abstand außerhalb des vorbestimmten Bereiches liegt.

14. Verwendung nach einem der Ansprüche 6 bis 13, **dadurch gekennzeichnet, dass** eine Vielzahl von Replikaten der Mikroorganismen-Suspensionen auf den Probenträger aufgebracht wird.

## Claims

1. Microbial test standard for use in infrared spectrometry which has at least two resealable vessels which are liquid-tight when closed, each of which contains a predefined amount of dried biomass of a microorganism, where the microorganisms in the different vessels differ in at least one characteristic and the difference manifests itself in a predefined intermicrobial spectral distance,
**characterized in that**
the dried biomass is sterilized.

2. The microbial test standard according to Claim 1, wherein the at least one characteristic is selected from the group: species, subspecies, strain, serovar, pathovar, toxivar and variety.

3. The microbial test standard according to Claim 1 or 2, wherein the dried biomass is comprised of vacuum-dried pellets.

4. The microbial test standard according to one of the Claims 1 to 3, wherein the microorganisms belong to different strains of one bacterial species.

5. The microbial test standard according to one of the Claims 1 to 4, wherein the vessels have screw caps.

6. Use of the microbial test standard according to one of the Claims 1 to 5, comprising:
- Provision of a sample support for infrared spectrometry which has an array of sample spots,
- Re-suspension of the biomass in the vessels by adding a solvent,
- Removal of a predetermined quantity of the suspension from each vessel, which is then deposited on a predetermined number of sample spots,
- Drying of the suspension on the sample spots,
- Acquisition of infrared spectra from the sample spots;
- Calculation of intermicrobial spectral distances between spectral signatures in the infrared spectra of the different sample spots; and
- Determination of whether the spectral distances are outside a predetermined range.

7. The use according to Claim 6, wherein the spectral distances are computed from the microbial spectral signatures in a predetermined wave number range and correspond to a predetermined distance.

8. The use according to Claim 7, wherein the predetermined wave number range is between around 1,300 and 800 cm⁻¹.

9. The use according to one the Claims 6 to 8, wherein the intramicrobial spectral distances between the spectral signatures in the infrared spectra of the different sample spots are computed to examine the spectral reproducibility.

10. The use according to Claim 9, wherein the predetermined range of the intramicrobial spectral distance extends to a maximum distance A, which is a measure for the reproducibility of the acquisitions, and the predetermined range of the intermicrobial spectral distance extends from a minimum distance B, which is a measure for the spectral specificity in the acquisitions.

11. The use according to one of the Claims 6 to 10, wherein the solvent for the re-suspension contains distilled and/or deionized water.

12. The use according to one of the Claims 6 to 11, wherein the re-suspension is promoted by agitating the vessels or by mix-pipetting.

13. The use according to one of the Claims 6 to 12, wherein a (warning) tag is attached to the IR spectra when a spectral distance is outside the predetermined range.

14. The use according to one of the Claims 6 to 13, wherein a plurality of replicates of the microorganism suspensions are applied to the sample support.

## Revendications

1. Norme d'essai microbien pour l'utilisation dans la spectrométrie infrarouge, qui présente au moins deux récipients refermables étanches aux liquides lorsqu'ils sont fermés, contenant chacun une quantité prédéfinie de biomasse séchée d'un micro-organisme, les micro-organismes des différents récipients présentant au moins une caractéristique différente et la différence se manifestant dans une distance spectrale intermicrobienne prédéfinie,
**caractérisé en ce que**
la biomasse séchée est stérilisée.

2. Norme d'essai microbien selon la revendication 1, dans laquelle l'au moins une caractéristique est choisie dans le groupe : espèce, sous-espèce, souche, sérovar, pathovar, toxivar et variété.

3. Norme d'essai microbien selon la revendication 1 ou 2, dans laquelle la biomasse séchée est constituée de granulés séchés sous vide.

4. Norme d'essai microbien selon l'une des revendications 1 à 3, dans laquelle les micro-organismes appartiennent à des souches différentes d'une espèce bactérienne.

5. Norme d'essai microbien selon l'une des revendications 1 à 4, dans laquelle les récipients sont munis de bouchons à vis.

6. Utilisation de la norme d'essai microbien selon l'une des revendications 1 à 5, comprenant :
- Fourniture d'un support d'échantillons pour la spectrométrie infrarouge avec une série de points d'échantillonnage,
- Remise en suspension de la biomasse dans les récipients par addition d'un solvant,
- Prélèvement d'une quantité prédéterminée de la suspension de chaque récipient et son application sur un nombre prédéterminé de points d'échantillonnage,
- Séchage de la suspension sur les points d'échantillonnage,
- Acquisition de spectres infrarouges à partir des points d'échantillonnage ;
- Calcul des distances spectrales intermicrobiennes entre les signatures spectrales dans les spectres infrarouges des différents points d'échantillonnage ; et
- Détermination de si les distances spectrales se situent en dehors d'une plage prédéterminée.

7. Utilisation selon la revendication 6, dans laquelle les distances spectrales sont calculées à partir des signatures spectrales microbiennes dans une plage de nombres d'ondes prédéterminée et correspondent à une distance prédéterminée.

8. Utilisation selon la revendication 7, dans laquelle la plage de nombre d'ondes prédéterminée est comprise entre environ 1.300 et 800 cm⁻¹.

9. Utilisation selon l'une des revendications 6 à 8, dans laquelle les distances spectrales intramicrobiennes entre les signatures spectrales dans les spectres infrarouges des différents points d'échantillonnage sont calculées pour examiner la reproductibilité spectrale.

10. Utilisation selon la revendication 9, dans laquelle la plage prédéterminée de la distance spectrale intramicrobienne s'étend jusqu'à une distance maximale A, qui est une mesure pour la reproductibilité des acquisitions, et la plage prédéterminée de la distance spectrale intermicrobienne s'étend depuis une distance minimale B, qui est une mesure pour la spécificité spectrale dans les acquisitions.

11. Utilisation selon l'une des revendications 6 à 10, dans laquelle le solvant pour la remise en suspension contient de l'eau distillée et/ou désionisée.

12. Utilisation selon l'une des revendications 6 à 11, dans laquelle la remise en suspension est favorisée par agitation des récipients ou par mélange-pipetting.

13. Utilisation selon l'une des revendications 6 à 12, dans laquelle une étiquette (d'avertissement) est attachée aux spectres IR lorsqu'une distance spectrale est en dehors de la plage prédéterminée.

14. Utilisation selon l'une des revendications 6 à 13, dans laquelle une pluralité de réplicats des suspensions de microorganisme est appliquée sur le support d'échantillons.
